# EUROPÄISCHE PATENTSCHRIFT

(11) **EP 1 446 070 B1**
(45) Veröffentlichungstag und Bekanntmachung des Hinweises auf die Patenterteilung: **03.06.2009**
(21) Anmeldenummer: 02779217.5
(22) Anmeldetag: 13.11.2002
(51) Int. Cl.: A61F 2/04, A61L 27/38

(54) **BIOARTIFIZIELLES TRACHEA-IMPLANTAT UND VERFAHREN ZU SEINER HERSTELLUNG**
BIO-ARTIFICIAL TRACHEA IMPLANT AND A METHOD FOR PRODUCING THE SAME
IMPLANT DE TRACHEE BIOARTIFICIEL ET SON PROCEDE DE PREPARATION

(30) Priorität: 20.11.2001 DE 10156561
(43) Veröffentlichungstag der Anmeldung: 18.08.2004
(73) Patentinhaber: corLife GbR, 30625 Hannover (DE)
(72) Erfinder: BRENNING, Ole, H., 30173 Hannover (DE); HAVERICH, Axel, 30916 Isernhagen (DE)
(74) Vertreter: Lins, Martina
(86) Internationale Anmeldenummer: PCT/DE2002/004214
(87) Internationale Veröffentlichungsnummer: WO 2003/045277

(56) Entgegenhaltungen:
- WO-A-00/15765
- WO-A-00/59618
- WO-A-99/62424
- WO-A-02/064179
- US-A1- 2001 039 047

## Beschreibung

Die Erfindung betrifft ein bioartifizielles Trachea-Implantat und ein Verfahren zu seiner Herstellung.

Unter einem "bioartifiziellen" Implantat verstehen wir hier ein mit den Mitteln des sogenannten "tissue engineering" gewonnenes mit technischen Mitteln nachgebildetes Gewebe aus biologischen Materialien. Im weiteren Sinne werden darunter hier auch solche Konstrukte gefasst, in die künstliche, nicht biologische Materialien (Kunststoffe, Polymere) mit eingearbeitet sind, jedoch sofern sie nicht biologisch abbaubar sind bevorzugt an vollständig von biologischen Materialien verdeckter Position oder an einer Position, an der keine ungünstigen immunologischen Reaktionen auf das künstliche Material zu erwarten sind, wie z.B. an vom Körper nach außen oder zum Verdauungstrakt gerichteten Stellen.

Als Grundlage für das bioartifizielle Implantat oder Gewebe werden biologische Matrixsubstanzen, in der Regel Kollagen, oder biologisch verträgliche, ggf. biologisch abbaubare Kunststoffe verwendet; diese können in eine Struktur gepresst oder gegossen werden. Unter einer biologischen Gewebematrix wird eine solche verstanden, die nach Azellularisierung eines durch Entnahme aus einem Körper gewonnenen natürlichen biologischen Materials als "Gerüst" mit natürlicher Struktur zurückbleibt. Die verschiedenen Matrices werden anschließend - gegebenenfalls nach Zwischenlagerung, Einfrieren oder sonstigen Verarbeitungsschritten - mit lebensfähigen Zellen besiedelt (rebesiedelt). Die verschiedenen für die Besiedlung benötigten Zellen werden häufig und wenn möglich aus autologen Zellen expandiert und in vitro kultiviert.

Gelegentlich werden als Matrix auch biologisch verträgliche Kunststoffe verwendet, die durch Besiedlung mit körpereigenen Zellen maskiert werden, um eine Abstoßung zu vermeiden. Wenn biologisch abbaubare Kunststoffe verwendet werden, hofft man, dass diese im Körper mit der Zeit zu natürlichem körpereigenem Material umgebildet werden (Remodelling). Diese Versuche sind jedoch häufig unbefriedigend.

Die Erfindung bezieht sich speziell auf ein bioartifizielles Trachea-Implantat.

Zahlreiche Ursachen erfordern eine Resektion und Rekonstruktion der Trachea. Hierunter fallen kongenitale Fehlbildungen, wie die langstreckige Trachealstenose, die Agenesie oder Atresie, tracheo-ösophageale Fisteln oder Traumata im Sinne eines Intubationstraumas oder nach Unfällen. Ferner machen primäre und sekundäre Tumoren (Ösophagus CA, Schilddrüsen CA) im Rahmen der Tumorchirurgie eine angemessene Resektion notwendig, die heute ausschließlich mit Re-Anastomosierungen der Trachea einhergehen (Backer CL, Mavroudis C, Dunham ME, Holinger L., Intermediate-term results of the free tracheal autograft for long segment longenital tracheal stenosis. J Pediatr Surg 2000 Jun; 35(6): 813-8; discussion 818-9).

Insbesondere die Carina-Resektion weist die höchste postoperative Mortalität in der Thoraxchirurgie auf. Etwa 17 % der Patienten versterben intraoperativ, bei ca. 39 % der Patienten treten postoperative Komplikationen auf, die in etwa 43 % der Fälle zum Tode führen. Zu den häufigsten Komplikationen zählt die Insuffizienz oder Stenose der Anastomose (17,2 %). Das 5-Jahresüberleben liegt bei ca. 42 % (Mitchell 2001). Der wesentliche Grund für die Komplikationen ist die hohe Spannung, die an der Trachea-Anastomose entsteht. Mit zunehmender Länge des zu reserzierenden Trachealsegments steigt die Spannung an der Anastomose und sinkt der Operationserfolg.

Aus diesem Grund sind Resektionen auf unter 50 % der Trachealänge (max. ca. 4-6 cm) beschränkt. Hierdurch wird die Indikation zum operativen Eingriff deutlich limitiert. Durch zahlreiche Ausschlusskriterien entgeht einem großen Teil der Patienten die Möglichkeit einer chirurgischen Intervention. Für die überwiegende Mehrzahl Betroffener stellt die Verfügbarkeit eines geeigneten Trachealersatzes somit eine lebenswichtige Therapieoption dar.

Zahlreiche Versuche sind unternommen worden, die Trachea mittels synthetischer Polypropylen-Gewebe (Marlix mesh tubes) (Sekine T, Nakamura T, Liu Y, Ueda H, Matsumoto K, Shimizu Y. Collagen coated Y-shaped prosthesis for carinal replacement promotes regeneration of the tracheal epithelium. ASAIO J 2000 Jul-Aug; 46(4): 421-5; Sekine T, Nakamura T, Shimizu Y, Liu Y, Ueda H, Matsumoto K, Experimental carinal replacement with an Y-shaped collagen-conjugated prosthesis. J Thorac Cardiovasc Surg 2000 Jun; 48(3): 125-9) oder biologischen Conduits (z.B. Aortensegmente, kryokonservierte Homografts) (Kunachak S, Kulapaditharom B, Vajaradul Y, Rochanawutanon M, Cryopreserved, irradiated tracheal homograft transplanation for laryngotracheal reconstruction in human beings. Otolaryngol Head Neck Surg 2000 Jun; 122(6):911-6) zu versorgen, jedoch fand bis heute keine Methode klinische Akzeptanz. Bei allen diesen Verfahren treten Nekrosen im Bereich der Anastomosen und somit Luftlecks auf, da diese Implantate nicht mit den patienteneigenen Gefäßen verbunden werden konnten.

Für den Ersatz von Trachealgewebe nach notwendigen Resektionen steht somit derzeit kein Implantat zur Verfügung. Es besteht ein dringendes Bedürfnis an Implantaten, mit denen die postoperativen Komplikationen vermieden und die bislang hohe intraoperative Mortalität verringert werden könnte.

Aus der WO 00/15765 A ist ein intestinales Submucosa-Gewebe als Zellkultursubstrat bekannt. Bevorzugt wird Tunica submucosa. Es können verschiedene Zellen aufgesiedelt werden, insbesondere Epithelzellen.

Aus der WO 00/59618 A ist ein Verfahren zur Besiedlung von Substraten mit biologischen Zellen bekannt, bei dem ein vorzugsweise tubuläres Substrat in eine Besiedlungsvorrichtung eingespannt und nach bestimmten Vorgaben von Zellsuspension umspült wird.

Die WO 99/62424 A offenbart ein Trachea-Implantat, bei dem vorgesehen ist, dass luminale Zellen auf eine tubuläre azellularisierte Kollagenmatrix aufgebracht werden. Dies kann auch mehrschichtig mit unterschiedlichen Zelltypen geschehen.

Der Erfindung liegt daher die Aufgabe zugrunde, ein bioartifizielles Trachea-Implantat und ein Verfahren zu seiner Herstellung zur Verfügung zu stellen, das die bei bisherigen Implantaten bestehenden Nachteile vermeidet und eine naturähnliche Anbindung und Versorgung des Implantats am Implantationsort ermöglicht.

Erfindungsgemäß wird diese Aufgabe gelöst durch das Verfahren nach Anspruch 1, nämlich ein Verfahren zur Herstellung eines bioartifiziellen Tracheaimplants, bei welchem lebensfähige Zellen mit Stützfunktion zwischen einer inneren, röhren- oder schlauchförmigen biologischen oder künstlichen Matrix und einer koaxial um diese angeordneten äußeren, mit einer Gefäßversorgung ausgestatteten
a) azellularisierten oder teilazellularisierten,
b) azellularisierten oder teilazellularisierten und wenigstens teilweise rebesiedelten oder
c) nativen tubulären Gewebematrix eingebracht werden, wobei die innere Matrix so innerhalb der äußeren Gewebematrix angeordnet wird, dass zwischen beiden ein ringförmiger bzw. schlauchförmiger, tubulärer Zwischenraum bleibt oder leicht gebildet werden kann, in den die Zellen mit Stützfunktion eingebracht und bis zur Bildung eines Zellverbandes zwischen den Matrices kultiviert werden.

Dies kann unter üblichen Kulturbedingungen bei 37 °C im Inkubator geschehen.

Weiterhin wird die Aufgabe gelöst durch ein bioartifizielles Trachea-Implantat nach Anspruch 15.

Bei der äußeren tubulären Gewebematrix handelt es sich erfindungsgemäß um eine einem Spenderkörper entnommene allogene, xenogene und autologe a) azellularisierte oder teilazellularisierte oder b) azellularisierte oder teilazellularisierte und wenigstens teilweise rebesiedelte oder c) native tubuläre, d.h. rohr- oder schlauchförmige, biologische vaskularisierte Gewebematrix, die vorzugsweise aus dem Magen-Darm-Trakt stammt und insbesondere dem Jejunum oder Ileum mit anhängendem, anschlussfähigem Gefäßast entnommen worden ist. Eine solche Matrix ist aus der WO 02/064179 A2 bekannt.

Wenn möglich sollte das für die äußere tubuläre Gewebematrix isolierte Gewebe autolog sein, d.h. dem Patienten entnommen sein, der später das Trachea-Implantat erhalten soll. Alternativ kommen allogene Materialien von menschlichen Organspendern oder auch xenogene Gewebematrices in Frage.

Die äußere tubuläre Gewebematrix besitzt eine primäre Gefäßversorgung, deren Struktur auch bei einer etwaigen Azellularisierung - die, wie nachfolgend noch beschrieben wird, nicht in jedem Falle erforderlich ist - erhalten bleibt. Bei der Isolation des Gewebes wird wenigstens ein funktionsfähiger Gefäßast, der auch ursprünglich (d.h. primär) mit dem Gewebe verbunden ist, zusammen mit dem Gewebe entnommen und mit diesem präpariert. Der wenigstens eine Gefäßast oder Gefäßbaum kann in besonders bevorzugter Ausführung so ausgebildet - sein, dass er mit einem intramuralen, d. h. die Wandschichten der Gewebematrix durchziehenden, Kapillarnetz verbunden ist. Es kann sich um ein klein-, mittel- oder größerlumiges Gefäßnetz handeln, wobei darauf zu achten ist, dass der Gefäßstiel chirurgisch an das Blutgefäßsystem des Implantat-Empfängers angeschlossen (anastomosiert) werden kann.

Der Gefäßast oder -baum besteht im allgemeinen aus einer zuführenden Arterie und einer abführenden Vene. Die Gewebematrix kann auch mit bis zu 6, vorzugsweise 2 bis 5 Gefäßästen verbunden sein. Besonders bevorzugt ist der Gefäßast mit einem dazugehörigen Netzwerk aus kleinlumigen Gefäßen versehen. Die im Einzelfall zu wählende Anzahl von Gefäßästen hängt u.a. von der Länge und Größe des Implantates ab. Das gesamte vorstehend beschriebene System von Blutgefäßen stellt die Gefäßversorgung der äußeren tubulären biologischen Gewebematrix dar.

Die Azellularisierung der bei diesem Verfahren verwendeten biologischen Matrices kann mit dem Fachmann hinlänglich bekannten Verfahren, d.h. mit allen bekannten Mitteln, wie chemischen, biochemischen, physikalischen, mechanischen oder sonstigen Verfahren, durchgeführt werden und braucht daher hier nicht gesondert beschrieben zu werden. Häufig erfolgt die Azellularisierung durch enzymatischen Zellverdau, bzw. mit schonenden chemischen Mitteln in einer Lösung, in die die Matrix eingelegt wird. Eine zumindest teilweise mechanische Ablösung der Zellen, vor allem oberer Schichten, ist möglich.

Bei Verwendung allogener oder xenogener Matrices werden Matrix und Gefäßäste vorzugsweise vollständig azellularisiert. Bei Verwendung einer autologen Matrix dagegen, in der Regel einer patienteneigenen Darmmatrix, kann eine Azellularisierung mit anschließender autologer Rebesiedlung in Einzelfällen unterbleiben oder durch Co-Kultur erfolgen. Dadurch kann die Besiedlung des Kapillarnetzes und des Gefäßsystems mit den Gefäßästen praktisch vollständig erhalten bleiben. Die geeigneten Azellularisierungsverfahren kann der Fachmann zweckentsprechend auswählen. Die nach teilweiser oder vollständiger Azellularisierung erhaltene Gewebematrix kann anschließend mit ebenfalls dem Fachmann bekannten Besiedlungsverfahren mit biologischen, lebensfähigen , d.h. zu Anwachsen und weiterem Wachstum befähigten, Zellen besiedelt werden.

Als innere Matrix kann eine Röhre aus biokompatiblem Material oder eine azellularisierte oder teilazellularisierte, ggf. wenigstens teilweise rebesiedelte, oder native röhrenförmige Gewebematrix verwendet werden. Sofern es sich um ein biologisches Material handelt, gilt für die Azellularisierung und Rebesiedlung grundsätzlich das für die äußere tubuläre Matrix gesagte. Alternativ, kann ein biokompatibles Material, z.B. aus Kunststoff verwendet werden. In Frage kommen u.a. Röhren aus Kunstfasergeweben, in die Zellen beidseitig gut einwachsen können.

Alternativ können auch glatte Röhren oder Folien mit vorzugsweise abweisender Oberfläche als innere Stützmatrix verwendet werden, wobei diese nach Abschluss des Verfahrens wieder entfernt werden kann. Auf der inneren Implantat-Oberfläche liegen dann nach Abschluss des Verfahrens die vorzugsweise autologen Zellen mit Stützfunktion frei. Diese können anschließend mit einer weiteren Deckschicht besiedelt werden, beispielsweise mit Flimmerepithel.

Die innere, biologische oder künstliche röhrenförmige Matrix, die einen kleineren Querschnittsdurchmesser hat als die äußere Gewebematrix (die mit Gefäßen versorgt ist) wird für das Verfahren so innerhalb der äußeren tubulären Gewebematrix angeordnet, dass zwischen beiden ein ring- bzw. wiederum schlauchförmiger, tubutärer Zwischenraum bleibt oder leicht gebildet werden kann, in den nun Zellen mit Stützfunktion eingebracht werden.

Unter "Zellen mit Stützfunktion" sind im Sinne dieser Erfindung solche Zellen zu verstehen, die auch natürlicherweise im Körper eine Stützfunktion ausüben (differenzierte Zellen eines Stützgewebes), in erster Linie Knorpelzellen - wie sie in natürlicher Trachea vorhanden sind. Die Knorpelzellen können auch unter Zumischung anderer Zellen mit Stützfunktion, wie z.B. Knochenzellen, verwendet werden. Hierdurch kann die Festigkeit des Implantats variiert werden. Vorzugsweise werden Knorpelzellen (Chondrozyten) verwendet, weiter vorzugsweise aus Rippenknorpel. Es können auch zu den gewünschten Zellen entwickelbare Vorläuferzellen, vorzugsweise mesenchymale Stammzellen, eingesetzt werden, die im Laufe der Kultivierung innerhalb dieses Verfahrens an Ort und Stelle zu den gewünschten Zellen mit Stützfunktion ausreifen.

Für das Einbringen zwischen die innere röhren- oder schlauchförmige Matrix und die äußere mit der Gefäßversorgung ausgestattete Matrix können die Zellen mit Stützfunktion wie üblich in Suspension in Nährlösung oder Blut vorliegen, oder aber in einer Zubereitung mit biologischen oder synthetischen Hilfsstoffen, wie z.B. Fibrinkleber, Kollagen usw. und/oder weiteren Zellen. Die Zubereitung oder die Suspension kann eine viskose oder pastöse Konsistenz haben und beispielsweise Gelbildner und/oder Polymere enthalten.

Das Einbringen erfolgt mit üblichen Mitteln, beispielsweise durch Einspritzen. In einer bevorzugten Ausführungsform kann die äußere Gewebematrix auf der inneren Matrix vor dem Einbringen der Zellen mit Stützfunktion mehrfach ringförmig abgebunden werden. Dies geschieht beispielsweise durch Schnüre oder Schläuche (Abbinder) von außen. Die Zellen werden danach - wie oben beschrieben in Nährlösung oder einer Zubereitung - in einen oder vorzugsweise mehrere ringförmige Zwischenräume zwischen äußerer und innerer Matrix eingespritzt.

Zusätzlich können weitere Zellen gemeinsam mit den Zellen mit Stützfunktion oder vor oder nach dem Einbringen der Zellen mit Stützfunktion in den ringförmigen Zwischenraum eingebracht werden. Diese oder andere weitere Zellen können auch in zusätzlichen Schritten außen auf die äußere Gewebematrix mit dem außenliegenden wenigstens einen Gefäßast und/oder innen auf die Innenseite der inneren röhrenförmigen Matrix (luminal) aufgebracht und aufgesiedelt werden.

Als weitere Zellen können je nach Bedarf die verschiedensten Zellen verwendet werden, insbesondere Fibroblasten und/oder Mesenchymalzellen, wie glatte Muskelzellen, Endothelzellen oder Epithelzellen. Die für die Besiedlung ausgewählten Zellen können autologe, allogene oder xenogene Zellen sein, äußerst vorzugsweise jedoch autologe bezogen auf den späteren Implantat-Empfänger. Es ist vorteilhaft, bereichsweise und/oder in mehreren Schichten mit verschiedenen Zellen zu besiedeln, so wie dies dem morphologischen Aufbau und der Funktion des natürlichen Trachealgewebes entspricht, welches dem Fachmann bekannt ist. Zu nennen ist hier auch die Besiedlung der inneren/luminalen Schicht mit Flimmerepithel.
Auf die Rebesiedlung von außen kann verzichtet werden, wenn die Matrix nativ und autolog ist und vom Körper nach Implantation selbst umgebildet werden soll oder azellularisiert ist und vom Körper nach Implantation selbst besiedelt werden soll.

Auf die Rebesiedlung von innen kann verzichtet werden, wenn die innere Matrix künstlich ist, xenogen oder allogen und azellularisiert, oder autolog.
Ob auf die in vitro Besiedlung verzichtet wird, kann der Fachmann nach den Anforderungen des Einzelfalls entscheiden, eine Rebesiedlung mit autologen Zellen wird jedoch im allgemeinen zu empfehlen sein.

Falls erwünscht können Mischungen verschiedener Zellarten verwendet werden. Die Zellen können innerhalb von Zellzubereitungen mit Hilfsstoffen vermengt aufgegeben werden. Die Zellen können auch vermengt mit Polymeren, wie Polycarbonaten, Polylactaten usw. verwendet werden. Insbesondere können Kollagene oder z.B. Fibrin(-kleber) verwendet werden.

Bei Verwendung einer allogenen oder xenogenen Gewebematrix wird der wenigstens eine Gefäßast vorzugsweise mit Endothelzellen besiedelt. Falls die innere Matrix ebenfalls biologisch durch Gewebeentnahme erhalten wurde, werden im Inneren (luminal) vorzugsweise autologe vaskuläre Endothelzellen aufgesiedelt.

Die für eine Besiedlung zu verwendenden Zellen stammen vorzugsweise aus biotisch gewonnenem Material, insbesondere vom vorgesehenen Implantat-Empfänger. Sie werden in vitro mit herkömmlichen Verfahren expandiert und mit im Stand der Technik bekannten Verfahren kultiviert.

Bereits während der Besiedlung können die aufgesiedelten Zellen mit Hilfe der angeschlossenen Gefäßversorgung, nämlich dem einen oder den mehreren Gefäßästen, mit Nährstoffen und Sauerstoff versorgt werden. Dadurch wird zunächst das Anwachsen und Einwachsen erleichtert und beschleunigt. Gerade bei größeren Zellzahlen und stärkeren Schichtdicken ermöglicht die Versorgung über das erfindungsgemäß vorhandene primäre Gefäßsystem erst eine ausreichende und dauerhafte Versorgung mit Nährstoffen, Mineralien und Sauerstoff. Ein frühzeitiges Absterben des Implantats durch Zelltod und degenerativen Umbau kann so verhindert werden. Nur so kann die Vitalität komplexer Zellverbände, Gewebe bzw. Organe erhalten werden. Durch das Besiedeln der Gewebematrix mit Zellen wird ein erfindungsgemäßes bioartifizielles, primär vaskularisiertes Trachea-Implantat erhalten. Dieses hat den Vorteil, dass es im Gegensatz zu herkömmlichen synthetischen, biologischen und bioartifiziellen Geweben primär vaskularisiert ist. Zeitgleich mit der Transplantation kann es daher an den Blutkreislauf des Transplantatempfängers angeschlossen werden. Die Zellen in allen Wandschichten des Implantates, also auch in den tiefergelegenen Arealen, können mit Nährstoffen versorgt werden und sterben nicht ab. Damit wird im Gegensatz zu allen bisher verfügbaren Trachea-Implantaten eine frühzeitige Degeneration, insbesondere eine Insuffizienz oder Stenose im Bereich der Anastomosen, vermieden und zusätzlich die langfristige Funktionsfähigkeit des Transplantats bzw. Implantats sichergestellt.

Wenn wenigstens ein vollständiger Gefäßast bereits bei Entnahme des Gewebes für die äußere Gewebematrix als intakter Gefäßstamm mit angeschlossenem intramuralen Kapillarnetz isoliert wird und während der weiteren Verfahrensschritte funktionsfähig gehalten wird, hat das erfindungsgemäße bioartifizielle, primär vaskularisierte Trachea-Implantat den Vorteil, bereits während der Herstellung die in vivo vorliegende biochemische zelluläre Zusammensetzung und morphologische geometrische Struktur zu besitzen. Damit werden das Einwachsen des Transplantats bzw. Implantats sowie eine den natürlichen Bedingungen und Anforderungen entsprechende Funktionsaufnahme im Empfänger erleichtert.

Ein weiterer großer Vorteil des erfindungsgemäßen bioartifiziellen, primär vaskularisierten Trachea-Implantats besteht darin, dass außer autologen Zellen auch autologes Gewebematerial verwendet werden kann (vorzugsweise Darmabschnitte des Empfängers), so dass eine Abstoßung vermieden wird.

Das erfindungsgemäße Trachea-Implantat kann eine ganze Trachea, z.B. auch mit Bifurkation, oder ein Teilstück sein. Der Trachea-Ersatz kann bedarfsgerecht für den Empfänger zugeschnitten sein, als längeres oder kurzeres Tracheastück oder beliebiger Trachea Abschnitt.

Da durch die hier beschriebene Technik die Möglichkeit besteht, die Länge des Trachea-Ersatzes anzupassen, können Zugkräfte an den Anastomosen vermieden werden, die ursächlich sind für das postoperative Auftreten von Stenosen oder Insuffizienzen im Bereich der Anastomosen.
Der erfindungsgemäße Trachea-Ersatz kann zur Behandlung jeder Erkrankung der Trachea (und des Bronchialbaums) eingesetzt werden, die mit z.B. Kompression oder Destruktion der Trachea einhergeht und die z.B. eine Resektion der Trachea oder eines Trachealsegments erforderlich macht.

Besonders bevorzugte Einsatzgebiete sind u.a. der Trachea- und Bronchialersatz bei pulmonalen und trachealen Fehlanlagen oder Fehlbildungen, wie z.B. langstreckige Trachealstenose, Agenesie oder Atresie, tracheo-ösophageale Fisteln. Bei Erkrankungen, nach Verletzungen oder Verlust durch Trauma, Tumor oder Entzündungen der Trachea oder eines Bronchus.

Durch die Erfindung wurde erstmals ein vitales vaskularisiertes Trachea-Implantat zur Verfügung gestellt, das sich gerade im Bereich der Anastomosen gut angleicht und damit Probleme, wie sie bei Einsatz von künstlichen Trachea-Prothesen entstehen, vermeidet. Das erfindungsgemäße Trachea-Implantat ermöglicht, selbst große Defekte spannungsfrei und langfristig suffizient zu rekonstruieren. Es ist zu erwarten, dass durch das neue Trachea-Implantat erstmals die intra- und postoperative Komplikationsrate (Morbidität) und damit verbunden die Mortalitätsrate bei trachealen Erkrankungen enorm gesenkt werden kann. Folgeoperationen nach Trachea-Operationen im Kindesalter werden durch eigenes Längenwachstum überflüssig gemacht. Die Erfindung besitzt daher auch erhebliche gesundheitsökonomische Bedeutung.

Durch die Erfindung eröffnen sich völlig neue Behandlungsmöglichkeiten von kurz- und insbesondere von langstreckigen Trhealstenosen, -neoplasien, -fisteln etc.

Im folgenden wird die Erfindung anhand eines Beispiels näher dargestellt. Das Beispiel dient rein illustrativen Zwecken. Dem auf dem Gebiet geschulten Fachmann werden daraus andere mögliche Ausführungsformen unmittelbar deutlich werden.

Zunächst wird das allgemeine Vorgehen anhand einer Skizze erläutert.

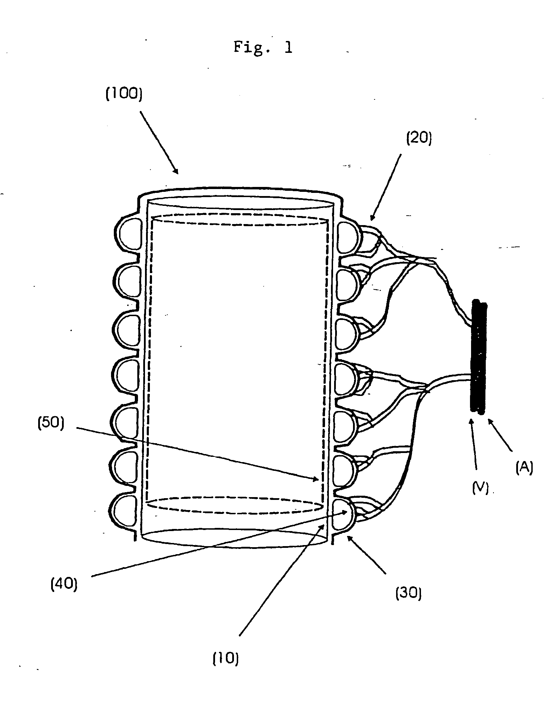

Figur 1 zeigt eine schematische Darstellung des Aufbaus des bioartifiziellen, primär vaskularisierten Trachea-Implantats im Längsschnitt.

Das im ganzen mit 100 bezeichnete Implantat zeigt eine innere, röhrenförmige Matrix 10, die nicht vaskularisiert ist und das Implantat während der Herstellung, zur inneren Oberfläche hin als eine Stützmatrix abschließt. Außen auf der inneren, röhrenförmigenmatrix 10 befindet sich, koaxial um die Matrix 10 angeordnet, eine äußere, mit einer Gefäßversorgung 20 ausgestattete Gewebematrix 30. Diese kann zu Beginn der Implantatherstellung auf der inneren Matrix 10 aufliegen, oder sie kann in einem geeigneten Reaktor im Abstand zur inneren Matrix 10 so eingespannt sein, dass sich ein ringförmiger Zwischenraum zwischen den beiden Matrizes 10 und 30 befindet. Die Gefäßversorgung 20 geht von einem eine Vene (V) und eine Arterie (A) umfassenden Gefäßast aus. Figur 1 zeigt weiter ringförmig aufgesiedelte Bereiche aus Zellen mit Stützfunktion 40, insbesondere Chondrozyten aus Rippenknorpel. Die ringförmigen Bereiche können beispielsweise erhalten werden, indem eine Stützzellen-(Chondrozyten-)Suspension nach Abbinden ringförmiger Bereiche zwischen die zunächst aufeinanderliegenden Matrizes 10 und 30 eingespritzt wird. Nach dem Einspritzen werden die Zellen auf der Matrix bzw. zwischen den Matrizes bis zur gewünschten Ringstärke bei 37 ° C inkubiert. Weiterhin ist bei dem schematisch gezeigten Ausführungsbeispiel auf der Innenseite der inneren, röhrenförmigen Matrix 10 ein Flimmerepithel 50 aufgebracht.

### Anwendungsbeispiel:

### Spendertiere:

Als Organspender für die Matrix-Gewinnung und zur Isolation von u.a. Knorpelzellen wurden weibliche Schweine im Alter von 3 Monaten verwendet.

Für die Matrix-Herstellung wurde ein Darmsegment mit anhängendem Gefäßast isoliert und mit 4 °C kalter Nebacetin-Lösung (1 g Nebacetin / 1000 ml NaCl 0,9%) intravasal und intraluminal perfundiert. Für den Transport wurde das vaskularisierte Darmsegment ebenfalls in 4 °C kalter Nebacetin-Lösung gelagert. Für die Chondrozyten-Gewinnung fand Rippenknorpel Verwendung. Hierzu wurden beidseits die zwei unteren Rippen entnommen und ebenfalls in 4 °C kalter Nebacetin-Lösung gelagert und transportiert. Die Organentnahmen sowie alle Operationen am Tier fanden stets unter den üblichen sterilen OP-Bedingungen statt.

### Zellisolierung:

Der Rippenknorpel wurde in 1 mm³-Stücke zerkleinert, in PBS-Lösung gewaschen und mit 1 mg/ml Typ-II-Kollagenase (Seromed, Berlin, Germany) über 16 h in einem Erlenmeyerkolben bei 37 °C im Wasserbad inkubiert. Durch Filtration durch ein Nylonsieb (Porengröße 200 m, Reichelt Chemie, Heidelberg) wurden Chondrozyten von unverdautem Knorpel getrennt. Inaktivierung der Kollagenase fand mittels Suspendieren in Zellmedium (HAM-F12, 10% FCS, 1 % Penicillin/Streptomycin) und Zentrifugieren statt. Zur Vitalitätsprüfung und Zellzahlbestimmung wurde die Trypan-Blau-Färbung verwendet.

### 3dimensionale Zellkultivierung:

In eine Kulturvorrichtung (Bioreaktor) wurde eine nicht vaskularisierte Matrixröhre gespannt, über die eine vaskularisierte Matrix wie oben erhalten gezogen wurde. In den spaltförmigen Zwischenraum wurden die Chondrozyten injiziert, suspendiert in Nährmedium oder Fibrinkleber mit ca. 10⁷ Zellen/ml. Die Kulturvorrichtung wurde mit Zellmedium (HAM-F12, 10 % FCS, 1 % Penicillin/Streptomycin) gefüllt, und es wurde bei 37 °C für 4 bis 6 Wochen inkubiert. Dabei wurde die Matrix außen und innen mit Zellmedium umspült.

### alternative Ausführungsformen:

Die Chondrozyten wurden auch im Gemisch mit Knochenmarkszellen oder Knochenzellen gegeben. Das Verhältnis ist frei einstellbar.

Nach Abschluss der Chondrozyten-Besiedlung wurden außen auf das Implantat (auf seiten des Gefäßasts) Endothelzellen aufgegeben und luminal vaskuläre Endothelzellen.

## Patentansprüche

1. Verfahren zur Herstellung eines bioartifiziellen Trachea-Implantats, bei welchem lebensfähige Zellen mit Stützfunktion (40) zwischen einer inneren, röhren- oder schlauchförmigen biologischen oder künstlichen Matrix (10) und einer koaxial um diese angeordneten äußeren, mit einer Gefäßversorgung (20) ausgestatteten
a) azellularisierten oder teilazellularisierten,
b) azellularisierten oder teilazellularisierten und wenigstens teilweise rebesiedelten oder
c) nativen
tubulären Gewebematrix (30) eingebracht werden, wobei die innere Matrix (10) so innerhalb der äußeren Gewebematrix (30) angeordnet wird, dass zwischen beiden ein ringförmiger bzw. schlauchförmiger, tubulärer Zwischenraum bleibt oder leicht gebildet werden kann, in den die Zellen mit Stützfunktion eingebracht und bis zur Bildung eines Zellverbandes zwischen den Matrices kultiviert werden.

2. Verfahren nach Anspruch 1, **dadurch gekennzeichnet, dass** als biologische lebensfähige Zellen mit Stützfunktion (40) Knorpelzellen (Chondrozyten), vorzugsweise Knorpelzellen aus Rippenknorpel, oder Knorpelzellen unter Zusatz anderer stützender Zellen, insbesondere Knochenzellen, und/oder hierzu entwickelbare Vorläuferzellen verwendet werden.

3. Verfahren nach Anspruch 1 oder 2, **dadurch gekennzeichnet, dass** die Zellen mit Stützfunktion (40) in einer Zubereitung zwischen die innere Matrix (10) und die äußere Gewebematrix (30) eingebracht werden.

4. Verfahren nach einem der Ansprüche 1 bis 3, **dadurch gekennzeichnet, dass** die äußere Gewebematrix (30) auf der inneren Matrix (10) vor dem Einbringen der Zellen mit Stützfunktion (40) mehrfach ringförmig abgebunden wird und dass die Zellen (40) in mehrere ringförmigen Zwischenräume zwischen äußerer und innerer Matrix eingebracht werden.

5. Verfahren nach einem der Ansprüche 1 bis 4, **dadurch gekennzeichnet, dass** die innere Matrix (10) nach Abschluss des Verfahrens entfernt wird.

6. Verfahren nach einem der Ansprüche 1 bis 5, **dadurch gekennzeichnet, dass** zusätzlich weitere Zellen gemeinsam mit den Zellen mit Stützfunktion (40) oder vor oder nach dem Einbringen der Zellen mit Stützfunktion zwischen die innere Matrix (10) und die äußere tubuläre Gewebematrix (30) eingebracht werden.

7. Verfahren nach einem der Ansprüche 1 bis 6, **dadurch gekennzeichnet, dass** zusätzlich weitere Zellen außen und/oder innen (luminal) auf das Trachea-Implantat aufgebracht werden.

8. Verfahren nach einem der Ansprüche 6 oder 7, **dadurch gekennzeichnet, dass** als weitere Zellen Fibroblasten und/oder Mesenchymalzellen, wie glatte Muskelzellen, Endothelzellen oder Epithelzellen verwendet-werden.

9. Verfahren nach einem der Ansprüche 1 bis 8, **dadurch gekennzeichnet, dass** Mischungen von Zellen und/oder Mischungen von Zellen mit Polymeren verwendet werden.

10. Verfahren nach einem der Ansprüche 1 bis 9, **dadurch gekennzeichnet, dass** für wenigstens eine der Zellarten autologe Zellen verwendet werden.

11. Verfahren nach einem der Ansprüche 1 bis 10, **dadurch gekennzeichnet, dass** die mit der Gefäßversorgung (20) ausgestattete Gewebematrix (30) aus dem Magen-Darm-Trakt stammt und vorzugsweise dem Jejunum oder Ileum entnommen ist.

12. Verfahren nach einem der Ansprüche 1 bis 11, **dadurch gekennzeichnet, dass** die Gefäßversorgung (20) wenigstens einen primär mit der Gewebematrix (30) verbundenen Gefäßast, insbesondere einen venösen (V) und einen arteriellen (A) Gefäßast oder Gefäßbaum, der weiter vorzugsweise mit einem intramuralen Kapillarnetz verbunden ist, aufweist, wobei der wenigstens eine Gefäßast vorzugsweise mit der verbundenen Gewebematrix (30) zusammen azellularisiert oder teilazellularisiert wurde.

13. Verfahren nach einem der Ansprüche 1 bis 12, **dadurch gekennzeichnet, dass** die Zellen mit Stützfunktion und/oder weitere aufgesiedelte Zellen über den wenigstens einen Gefäßast mit Nährstoffen versorgt werden.

14. Verfahren nach einem der Ansprüche 1 bis 13, **dadurch gekennzeichnet, dass** als innere, röhren- oder schlauchförmige, biologische oder künstliche Matrix eine Röhre aus biokompatiblem Material, eine schlauchförmige Folie oder eine azellularisierte oder teilazellularisierte, ggf. wenigstens teilweise rebesiedelte, oder native röhrenförmige Gewebematrix verwendet wird.

15. Bioartifizielles Trachea-Implantat, erhältlich durch ein Verfahren nach einem der Ansprüche 1 bis 14.

16. Bioartifizielles Trachea-Implantat mit einer inneren, röhren- oder schlauchförmigen biologischen oder künstlichen Matrix (10), einer äußeren, mit einer Gefäßversorgung (20) ausgestatteten a) azellularisierten oder teilazellularisierten, b) azellularisierten oder teilazellularisierten und wenigstens teilweise rebesiedelten, oder c) nativen tubulären Gewebematrix (30) sowie mit in wenigstens einem ringförmigen Zwischenraum zwischen den Matrizes (10;30) befindlichen lebensfähigen Zellen mit Stützfunktion (40).

17. Bioartifizielles Trachea-Implantat, nach Anspruch 16, **dadurch gekennzeichnet, dass** sich auf der nach innen gerichteten Oberfläche der inneren Matrix (10) Flimmerepithel befindet.

## Claims

1. Process for producing a bioartificial trachea implant, in which viable cells with supporting function (40) are introduced between an inner cylindrical or tubular biological or artificial matrix (10) and an outer
a) acellularised or partly acellularised,
b) acellularised or partly acellularised and at least partly recolonised or
c) native
tubular tissue matrix (30) which is disposed coaxially around the latter and is provided with a vascular supply (20), where the inner matrix (10) is disposed within the outer tissue matrix (30) in such a way that an annular or tubular space remains or can easily be formed between the two, into which space the cells with supporting function are introduced and are cultured until a cell assemblage forms between the matrices.

2. Process according to Claim 1, **characterized in that** cartilage cells (chondrocytes), preferably cartilage cells from costal cartilage, or cartilage cells with the addition of other supporting cells, especially bone cells, and/or progenitor cells which can be developed for this purpose, are used as biological viable cells with supporting function (40).

3. Process according to Claim 1 or 2, **characterized in that** the cells with supporting function (40) are introduced in a preparation between the inner matrix (10) and the outer tissue matrix (30).

4. Process according to any of Claims 1 to 3, **characterized in that** the outer tissue matrix (30) is multiply annularly ligated on the inner matrix (10) before the introduction of the cells with supporting function (40), and that the cells (40) are introduced into a plurality of annular spaces between outer and inner matrix.

5. Process according to any of Claims 1 to 4, **characterized in that** the inner matrix (10) is removed after completion of the process.

6. Process according to any of Claims 1 to 5, **characterized in that** additionally further cells are introduced together with the cells with supporting function (40) or before or after the introduction of the cells with supporting function between the inner matrix (10) and the outer tubular tissue matrix (30).

7. Process according to any of Claims 1 to 6, **characterized in that** additionally further cells are applied externally and/or internally (luminally) to the trachea implant.

8. Process according to either of Claims 6 or 7, **characterized in that** fibroblasts and/or mesenchymal cells such as smooth muscle cells, endothelial cells or epithelial cells are used as further cells.

9. Process according to any of Claims 1 to 8, **characterized in that** mixtures of cells and/or mixtures of cells with polymers are used.

10. Process according to any of Claims 1 to 9, **characterized in that** autologous cells are used for at least one of the cell types.

11. Process according to any of Claims 1 to 10, **characterized in that** the tissue matrix (30) provided with the vascular supply (20) is derived from the gastrointestinal tract and has been taken preferably from the jejunum or ileum.

12. Process according to any of Claims 1 to 11, **characterized in that** the vascular supply (20) includes at least one vascular branch connected primarily to the tissue matrix (30), in particular a venous (V) and an arterial (A) vascular branch or vascular tree, which is further preferably connected to an intramural capillary network, with the at least one vascular branch, preferably together with the connected tissue matrix (30), having been acellularised or partly acellularised.

13. Process according to any of Claims 1 to 12, **characterized in that** the cells with supporting function and/or further colonised cells are supplied with nutrients via the at least one vascular branch.

14. Process according to any of Claims 1 to 13, **characterized in that** a tube of biocompatible material, a tubular sheet or an acellularised or partly acellularised, optionally at least partly recolonised, or native cylindrical tissue matrix is used as inner, cylindrical or tubular, biological or artificial matrix.

15. Bioartificial trachea implant obtainable by a process according to any of Claims 1 to 14.

16. Bioartificial trachea implant with an inner, cylindrical or tubular biological or artificial matrix (10), with an outer a) acellularised or partly acellularised, b) acellularised or partly acellularised and at least partly recolonised, or c) native tubular tissue matrix (30) which is provided with a vascular supply (20), and with viable cells with supporting function (40) which are present in at least one annular space between the matrices (10; 30).

17. Bioartificial trachea implant according to Claim 16, **characterized in that** ciliated epithelium is present on the inwardly directed surface of the inner matrix (10).

## Revendications

1. Procédé de production d'un implant de trachée bioartificiel, dans lequel des cellules viables présentant une fonction d'appui (40) entre une matrice (10) biologique ou artificielle interne formant un tube ou un tuyau et une matrice de tissu (30) tubulaire externe sont placées au niveau co-axial autour de celle-ci, équipée d'une alimentation vasculaire (20)
a) acellularisée ou partiellement acellularisée,
b) acellularisée ou partiellement acellularisée et au moins partiellement re-colonisée, ou c) native,
la matrice interne (10) étant disposée à l'intérieur de la matrice de tissu externe (30) de telle sorte qu'entre les deux, un espace creux annulaire ou sous forme de tuyau tubulaire demeure ou puisse être facilement formé, dans lequel les cellules sont insérées avec une fonction d'appui et sont cultivées jusqu'à la formation d'une bande de cellules entre les matrices.

2. Procédé selon la revendication 1, **caractérisé en ce que** l'on utilise comme cellules viables biologiques présentant une fonction d'appui (40), des cellules cartilagineuses (chondrocytes), de préférence des cellules cartilagineuses de cartilage costal ou des cellules cartilagineuses en plus d'autres cellules d'appui, en particulier, des cellules osseuses et/ou des cellules précurseurs développées à cette fin.

3. Procédé selon la revendication 1 ou 2, **caractérisé en ce que** les cellules présentant une fonction d'appui (40) sont placées dans une préparation entre la matrice interne (10) et la matrice de tissu externe (30).

4. Procédé selon l'une des revendications 1 à 3, **caractérisé en ce que** la matrice de tissu externe (30) est liée circulairement à multiples reprises sur la matrice interne (10) avant insertion des cellules présentant une fonction d'appui (40) et que les cellules (40) sont insérées dans plusieurs espaces intermédiaires circulaires entre les matrices externe et interne.

5. Procédé selon l'une des revendications 1 à 4, **caractérisé en ce que** la matrice interne (10) est retirée à la fin du processus.

6. Procédé selon l'une des revendications 1 à 5, **caractérisé en ce que** d'autres cellules sont en outre insérées, conjointement avec les cellules présentant une fonction d'appui (40) avant ou après l'insertion des cellules présentant une fonction d'appui entre la matrice interne (10) et la matrice de tissu tubulaire externe (30).

7. Procédé selon l'une des revendications 1 à 6, **caractérisé en ce que** d'autres cellules sont en outre insérées à l'extérieur et/ou à l'intérieur (dans le lumen) d'un implant de trachée.

8. Procédé selon l'une des revendications 6 ou 7, **caractérisé en ce que** l'on utilise comme autres cellules, des fibroblastes et/ou des cellules mésenchymateuses telles que les cellules musculaires lisses, des cellules endothéliales ou des cellules épithéliales,

9. Procédé selon l'une des revendications 1 à 8, **caractérisé en ce que** l'on utilise des mélanges de cellules et/ou des mélanges de cellules avec des polymères.

10. Procédé selon l'une des revendications 1 à 9, **caractérisé en ce que** l'on utilise pour au moins l'un des types de cellules, des cellules autologues.

11. Procédé selon l'une des revendications 1 à 10, **caractérisé en ce que** la matrice de tissu (30) équipée d'une alimentation vasculaire (20) provient des voies gastro-intestinales, de préférence du jéjunum ou de l'iléum.

12. Procédé selon l'une des revendications 1 à 11, **caractérisé en ce que** l'alimentation vasculaire (20) présente au moins une branche vasculaire primaire liée à la matrice de tissu (30), en particulier une branche vasculaire artérielle (A) et veineuse (V) ou un arbre vasculaire qui est relié de préférence à un réseau capillaire intramural, au moins une branche vasculaire étant de préférence acellularisée ou partiellement acellularisée conjointement avec la matrice de tissu liée (30) .

13. Procédé selon l'une des revendications 1 à 12, **caractérisé en ce que** les cellules présentant une fonction d'appui et/ou d'autres cellules fixées sont alimentées par au moins une branche vasculaire avec des substances nutritives.

14. Procédé selon l'une des revendications 1 à 13, **caractérisé en ce que** l'on utilise comme matrice interne, biologique ou artificielle, en forme de tube ou de tuyau, un tube en matériau biocompatible, une feuille tubulaire ou une matrice de tissu acellularisée ou partiellement acellularisée, éventuellement au moins en partie re-colonisée, ou tubulaire native.

15. Implant de trachée bioartificiel, pouvant être obtenu par un procédé selon l'une des revendications 1 à 14.

16. Implant de trachée bioartificiel comportant une matrice (10) interne, biologique ou artificiel en forme de tube ou de tuyau, une matrice de tissu (30) externe équipée d'une alimentation vasculaire (20) a) acellularisée ou partiellement acellularisée, b) acellularisée ou partiellement acellularisée et au moins partiellement re-colonisée, ou c) native tubulaire et au moins des cellules viables présentant une fonction d'appui (40) se trouvant dans au moins un espace intermédiaire circulaire entre les matrices (10 ; 30).

17. Implant de trachée bioartificiel selon la revendication 16, **caractérisé en ce qu'**un épithélium cilié se trouve sur la surface de la matrice interne (10) dirigée vers l'intérieur.
